# EUROPEAN PATENT SPECIFICATION

(11) **EP 1 841 002 B1**
(45) Date of publication and mention of the grant of the patent: **20.05.2009**
(21) Application number: 06006905.1
(22) Date of filing: 31.03.2006
(51) Int. Cl.: H01M 10/42

(54) **Battery leakage detection system**
System zum Nachweis eines Lecks in einer Batterie
Systeme de detection de fuite dans une batterie

(43) Date of publication of application: 03.10.2007
(73) Proprietor: SONY DEUTSCHLAND GMBH, 10785 Berlin (DE); Sony Corporation, Tokyo 141-0001 (JP)
(72) Inventor: Vossmeyer, Tobias Stuttgart Technology Center, 70327 Stuttgart (DE); Joseph, Yvonne Stuttgart Technology Center, 70327 Stuttgart (DE); Yasuda, Akio Stuttgart Technology Center, 70327 Stuttgart (DE); Ogisu, Kenji, Tokyo 141-0001 (JP); Nishi, Yoshio, Tokyo 141-0001 (JP)
(74) Representative: Appelt, Christian W.

(56) References cited:
- US-A1- 2005 255 381
- PATENT ABSTRACTS OF JAPAN vol. 1998, no. 02, 30 January 1998 (1998-01-30) & JP 09 259898 A (EBARA KATSUO; YUASA CORP), 3 October 1997 (1997-10-03)
- S. A. HOOKER: "Nanotechnology Advantages Applied to Gas Sensor Development" 2002, BUSINESS COMMUNICATIONS CO, INC., NORWALK, CT USA , XP002393754 * page 1 - page 7 *

## Description

The present invention relates to a system for detection of chemical substances leaking from a battery.

Portable electronic devices like computers, mobile phones and audio/video equipment use primary, non-rechargeable or secondary, rechargeable batteries as power supply. Battery cells, and especially lithium ion battery cells used in rechargeable batteries, contain hazardous chemicals, which can become quite dangerous for a user if the battery shell becomes leaky. Such leakage of battery cells can be caused by material ageing, but also if the batteries are subjected to extreme environmental changes (e.g. temperature variations). Many attempts have been made to ensure the safe handling and usage of battery cells.

For example, secondary batteries are often embedded in battery packs. To avoid serious damage of the host equipment by chemical substances leaking from defective batteries, attempts have been made to construct the housing of the battery and the battery pack as good as possible. In addition, product and quality controls of the manufactured batteries are performed. Nevertheless a damage or malfunction of the batteries due to leakage cannot be excluded. Several approaches for the detection of leaking batteries have been made.

For example the use of a battery leakage sensing and warning system based on the electrical connection of electrodes of a sensor by liquid electrolyte has been disclosed in US 5824883. A detection system based on the reduction of the resistance of a sensor by liquid electrolyte is disclosed in DE 4220494.

In prior art systems, where leaks are detected by a contact between the liquid electrolyte of the battery and a sensing means, the disadvantage occurs that the sensing means must be arranged close to all locations of potential leaks in order to detect a leak if only a small quantity of electrolyte has leaked from the battery. Otherwise, if the sensing means is only arranged at a single point somewhere close to the battery, a leak in the battery which is not close to the sensing means will only be detected if a larger quantity of electrolyte has leaked from the battery which is sufficient to reach the sensing means. Known systems try to overcome this problem by using large sensitive areas, however, making the sensor more expensive and its installation more complicated.

This general problem may be overcome by gas sensors, where the exact location of the leak is less important since leaking electrolyte always has volatile components which diffuse towards the sensor rather quickly. The system described in JP 9259898 is based on the investigation of the gas phase surrounding the battery using a metal oxide semiconductor sensor.

The respective sensors known so far, however, need a high temperature for their operation, which again increases the risk potential of the battery system located close to the sensor and which furthermore requires a high operation power.

Improvements of gas sensor devices through nanoengineering are described in S.A. Hooker "Nanotechnology Advantages Applied to Gas Sensor Development", The Nanoparticles 2002 Conference Proceedings, Copyright @ Business Communications Co. Inc., Norwalk, CT USA.

Therefore, it is an object of the present invention to provide a highly efficient battery leakage detection system having a high sensitivity and a very low power consumption.

These object is achieved by a battery leakage detection system according to claim 1 and by a method for detecting a leakage of a battery according to claim 13.

Advantageous embodiments of the present invention are defined in the dependent claims.

According to the invention a battery leakage detection system is provided which is characterized therein that it comprises a gas sensor having a gas sensitive nanoparticle structure. This nanoparticle structure comprises according to one embodiment at least one nanoparticle.

The inventive sensor which is based on gas phase detection of chemicals does not require direct contact with the electrolyte or any visual inspection. Therefore, it may have a very small size. Especially in the case, where the nanoparticle structure comprises only one nanoparticle the sensor may be designed with very small dimensions. Moreover, the inventive system is fast, cheap to produce and very sensitive. Additionally, the system has a very little power consumption and has the advantage that it requires only a simple electrical signal transduction.

According to an embodiment the gas sensitive nanoparticle structure is a metal-nanoparticle/organic composite structure or a semi-conducting polymer structure or a polymer/carbon black composite structure or a combination of at least two of these structures. Those structures do offer a very high sensitivity for volatile chemicals.

According to a further embodiment the gas sensor is a sensor working on the basis of analyte induced changes of its conductance, capacitance, inductance, dielectric permittivity, polarization, impedance, heat capacity or temperature. Sensors of such kind are of great advantage, since they are very sensitive and do require only very little power consumption and do work at room temperature.

According to the present invention also a battery leakage detection system is provided which is characterized in that the system comprises at least one mass sensitive gas sensor, in particular a sensor comprising a quartz crystal microbalance, a surface acoustic wave device or a chemically sensitive field effect transistor. Those devices do comprise a very high sensitivity and do already respond to very small quantities of an analyte.

According to a further embodiment the system comprises at least one reference sensor for a sensor, said reference sensor and said sensor the reference sensor is related to comprising respective gas sensitive structures being isolated from each other. The use of a reference sensor has the advantage that environmental changes such as an increase or decrease of temperature or of humidity may be eliminated by the use of a reference sensor, thus further increasing the measurement sensitivity of the system.

According to a further preferred embodiment the reference sensor and the sensor are in contact for temperature exchange. Due to this embodiment temperature changes imposing drifts to the measurement result may be eliminated from the measurement since a ratio between the sensor used for detecting chemical substances and the reference sensor may be calculated in order to generate a baseline for the measurement. Furthermore, both sensors may be provided on the same substrate, thus facilitating the production process and the mounting of the sensor at a location e.g. in a battery housing in an electronic equipment which is to be monitored.

According to a further advantageous embodiment the system comprises a closed or tight housing, in particular a battery housing in which a gas sensor is arranged. Providing a closed or tight housing further increases the sensitivity of the system, since chemicals in the gas phase coming from a defective battery are hindered from diffusing further away from the battery and thus from the sensor.

A further preferred embodiment provides a further closed or tight housing in which a further gas sensor is arranged. In devices where two or more batteries are provided those may be located in separate closed or tight housings each comprising at least one sensor. Accordingly, one sensor may always serve as a reference sensor for the other sensor provided in the other housing.

According to a further preferred embodiment the system comprises a funnel for collecting volatile chemicals from a defective battery, a sensor chamber housing said sensor, a pump for pumping air to and/or drawing air past said sensor, and/or a pre-concentrator unit connected to each other. By combining one or several of the elements according to this embodiment, e.g. by a suited pipe system, a system for testing batteries during or after a production process for leaks may be provided.

Still another advantageous embodiment provides a means for conveying batteries to and from a test location provided in the system and/or means for automatically sorting out defective batteries. According to this embodiment a fully automatic test system for the batteries may be conceived.

According to yet another embodiment it is preferred to provide a battery leakage detection system in an electronic equipment. Such an electronic equipment may be preferably portable.

According to the invention also a method for detecting a leakage of a battery is provided, the method comprising the steps of providing a gas sensor having a gas sensitive nanoparticle structure close to a battery, the step of detecting analyte induced changes of a physical quantity such as the electrical conductance, capacitance, inductance, dielectric permittivity, polarization, impedance, heat capacity or temperature in said gas sensor indicating a defective battery. Using the inventive method comprising the steps mentioned a highly effective method consuming only very little power is provided.

According to a further advantageous embodiment of the invention the method furthermore comprises the steps of providing a pre-concentrator unit in front of said gas sensor; the step of bringing volatile chemicals from a defective battery in contact with said pre-concentrator unit; the step of applying a heat pulse to said pre-concentrator unit for desorbing volatile chemical compounds adsorbed to said pre-concentrator unit; and the step of bringing said desorbed volatile chemical compounds in contact with said gas sensor. Providing those steps the inventive method may be provided with even a still higher sensitivity.

According to yet another embodiment of the present invention the method further comprises the step of triggering an optical or acoustical signal in case an analyte induced change of the electrical conductance, capacitance, inductance, dielectric permittivity, polarization, impedance, heat capacity or temperature in said gas sensor is detected.

According to still another embodiment the method comprises the further step of automatically sorting out said defective battery.

Further features, advantages and characteristics of the present invention will now become apparent from the following description which in combination with the appended drawings describes preferred embodiments of the present invention.
- Fig. 1: shows a schematic drawing of a system for detection of chemical substances according to a preferred embodiment.
- Fig. 2A: shows a schematic drawing of a chemiresistor-type gas sensor.
- Fig. 2B: shows a schematic drawing of a sensor system comprised of two gas sensors.
- Fig. 3: shows a schematic drawing of a battery pack or battery housing divided in two compartments according to a preferred embodiment of the present invention.
- Fig. 4: shows a schematic drawing of a simple arrangement for testing batteries according to a preferred embodiment of the invention.
- Fig. 5: shows a drawing of a further configuration for testing batteries.
- Fig. 6: shows another configuration for testing batteries according to a further preferred embodiment of the invention.
- Fig. 7: shows a schematic drawing of a configuration for testing batteries consisting of two systems according to a further embodiment.
- Fig. 8: shows a schematic drawing of a configuration for testing batteries according to yet another embodiment.
- Fig. 9: shows a schematic drawing of a configuration for testing batteries according to another embodiment and similar to the arrangement in Fig. 6.
- Fig. 10: shows a chemiresistor device according to a preferred embodiment.
- Fig. 11 a), b) and c): show diagrams representing sensor responses to vapors of different electrolytes.

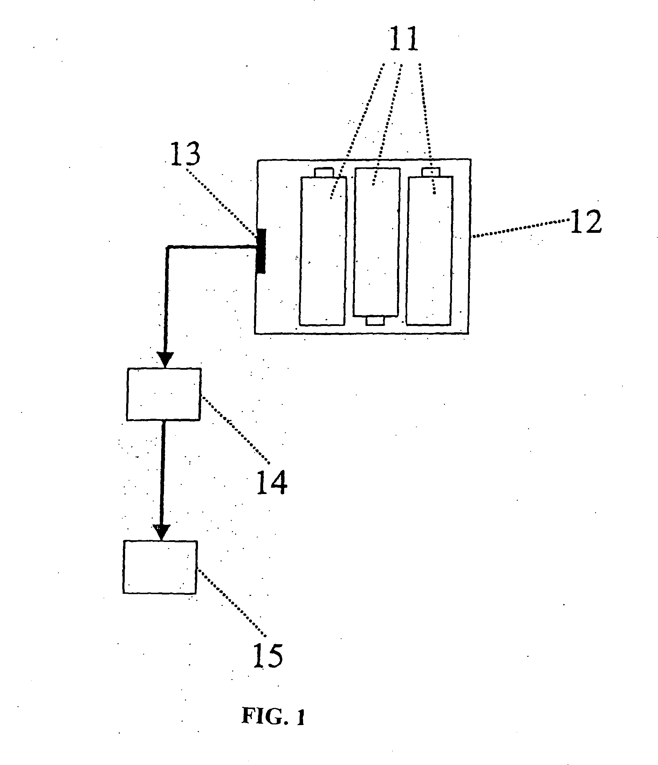

Figs. 1-3 give examples how the gas sensors can be employed in a battery housing or a battery pack. Those examples preferably relate to the application of the invention for monitoring batteries in electronic products.

Fig. 1 shows an arrangement according to a first embodiment. A gas sensor 13 is installed somewhere within a battery housing 12 or within a battery pack, respectively. As soon as a battery 11 starts to leak chemicals, volatile compounds diffuse to the location of the sensor 13 and trigger a sensor signal 14. The latter is used by a safety management system 15 to provide for example a message to the user of the product and/or to initiate a safety shutdown. The safety management system 15 may utilize an intranet or internet connection to send or receive sensor signals or to provide information about the battery status to a remote location. To minimise air circulation in the battery housing 12 and, thus to ensure reliable detection of a leaking battery 11, it is preferred that the battery housing 12 is closed or even gas tight.

Many types of gas sensors 13 are available, which can be used for the proposed invention. Such sensors may also be mass sensitive sensors based on quartz crystal microbalances (QCMs), or surface acoustic waves (SAW) devices. Other examples are sensors, which work on the basis of analyte induced changes of one or several of their physical or chemical properties such as conductance, capacitance, inductance, dielectric permittivity, polarisation, impedance, heat capacity or temperature. More specific examples are chemically sensitive field effect transistors (Chem-FETs). The sensors used in this invention may or may not be part of an integrated circuit.

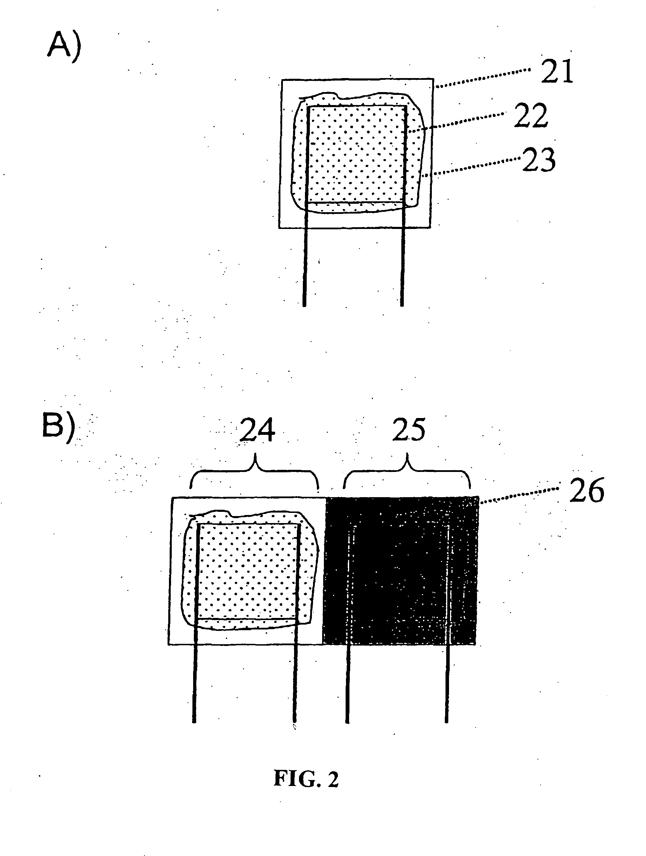

Fig. 2A shows preferred gas sensors to be used for the purpose of the invention. Fig. 2A shows a chemiresistor-type gas sensor. A sensitive film material 23 coated on a substrate 21 is contacted by two electrodes 22 to measure its electrical resistance. When the film is exposed to an analyte the change of its electrical resistance is used as the sensor signal. Many examples of film materials, which are used for chemiresistor-type sensors have been reported, which include: conducting and semi-conducting polymers, polymers/carbon black composite films, metal oxide semiconductors, carbon nanotubes, metal oxide nanofibres. In order to keep the power consumption as low as possible and to ensure safe operation, sensor coatings, which enable operation at room temperature, are preferred. Especially preferred are sensor coatings from metal-nanoparticle/organic composite materials.

Fig. 2B shows a more preferred arrangement of the sensor device. This device combines two sensors 24 and 25, one of which is coated with an inert material 26 (or otherwise encapsulated) so that the chemically sensitive surface is not exposed to the volatile chemicals in case of battery leakage. The coated sensor 25 acts as a reference sensor and is used to compensate for temperature drifts and/or aging of the sensor coating. To enable an efficient temperature-drift compensation it is important that both sensors 24, 25 are in good thermal contact with each other. Persons skilled in the art know such sensor arrangements, which include so called ratiometric sensors. The two sensors 24 and 25 can be part of a potential divider or a Wheatstone bridge arrangement to enable sensitive sensor readout. As sensor coatings any suitable, sensitive material may be used. Preferred sensor coatings may include those as described above with respect to Fig. 2A.

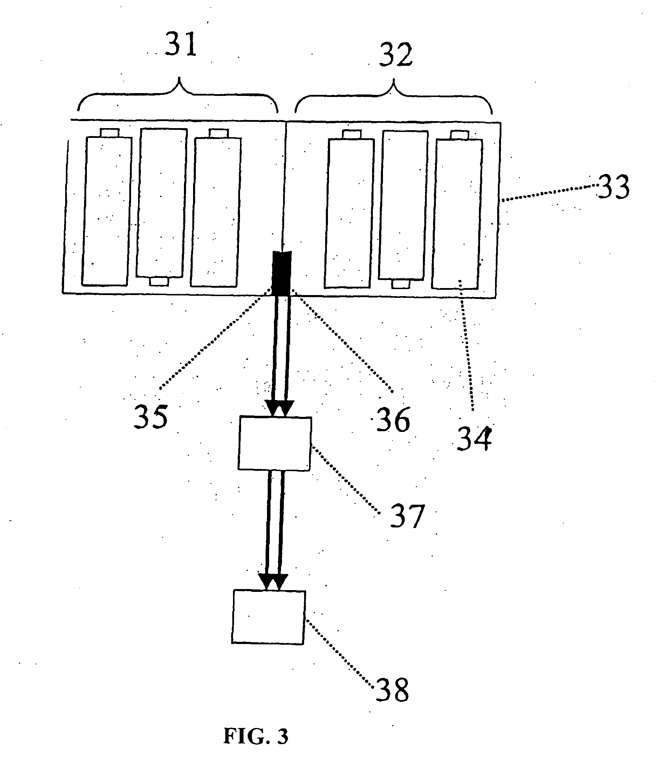

Fig. 3 shows a special arrangement. In this case the battery housing 22 or battery pack is divided into two compartments 31 and 32. These compartments are sufficiently sealed or may be even gas tight to minimize or exclude gas exchange between the two compartments 31 and 32 and with the outer environment. Within each compartment 31 and 32 there is one chemical sensor 35, 36, preferably of the same type and preferably comprising the same sensing material. Similar as in the case described above the signals of the two sensors 35, 36 are compared with each other, for example by monitoring the ratio of their electrical resistance. For compensating baseline drifts due to temperature fluctuations, both sensors 35, 36 are preferably in good thermal contact with each other. As described above the sensors 35, 36 may be part of a potential divider or a Wheatstone bridge arrangement to enable sensitive sensor readout. If in one compartment 31 a battery cell 34 starts leaking, volatile chemicals will trigger a signal of the sensor 35 located in that department, whereas the other sensor 36 remains unaffected. Thus, the ratio of the sensor resistances changes. This signal 37 is provided to the safety management system 38 for further processing the information, as described above.

Preferably the sensors 35, 36 used are chemiresistor-type sensors as shown and described with respect to Fig. 2A. Also a combination of the sensors shown in Fig. 2B and Fig. 2A is possible. Any suitable sensor material can be used as coating.

Instead of two compartments 31, 32 it is obvious that the battery housing or battery pack can be divided into more compartments, with each compartment equipped with one gas sensor.

Concerning the application of gas sensors for the detection of defective battery cells in the production process (i.e. for quality and/or product control) the following embodiments are preferred:

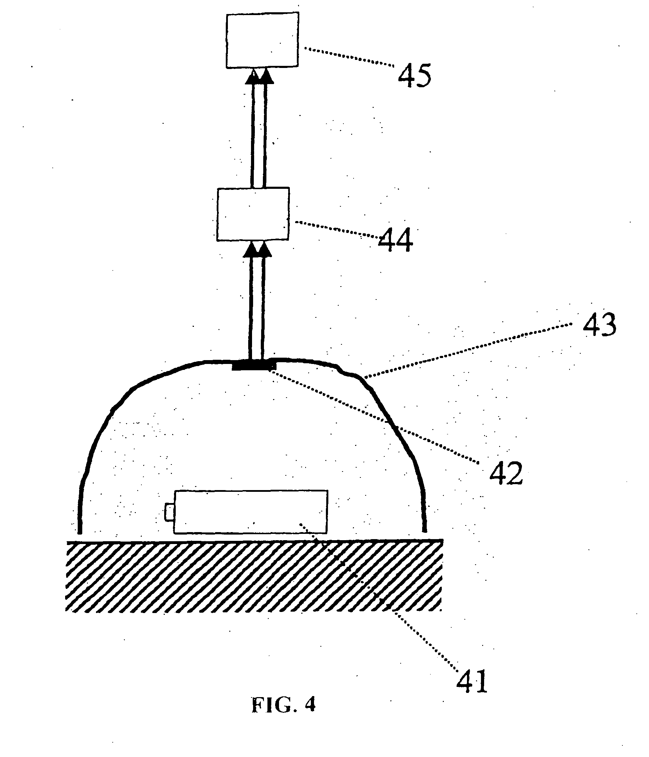

Figure 4 shows a simple arrangement for the quality control of battery cells. The system includes a cover 43, which comprises a gas sensor 42. For a leakage test the cover 43 is installed on the battery 41 to be tested. If the battery 41 has a leak the sensor signal 44 may trigger a robot system 45 to automatically sort out the defective battery or may trigger any optical or acoustical signal. The sensor 42 may be a single sensor or may also use a reference sensor as shown in Fig. 2B. If the reference sensor is located inside the cover it has to be encapsulated. If it is located outside the cover it may or may not be encapsulated. As pointed out above, the reference sensor and the sampling sensor are preferably in good thermal contact. Any suitable sensor material can be used as sensor coating. However, preferred are chemiresistor-type sensors which are operated at room temperature and which have been described above with respect to Fig. 2A.

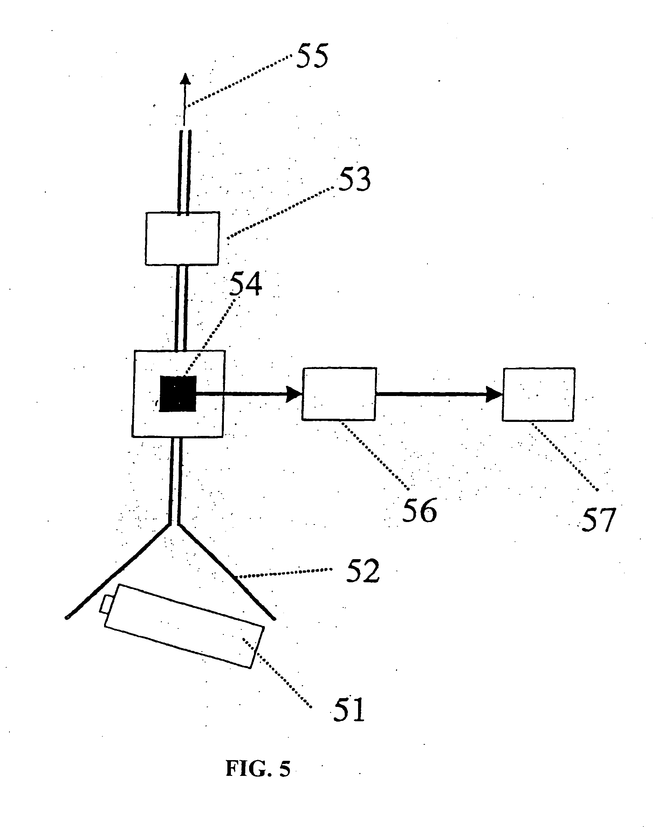

Fig. 5 shows a preferred sensor arrangement for the quality control of battery cells 51. The system comprises a funnel 52 for collecting volatile chemicals emitted from a defective battery cell 51. Behind the funnel a sensor chamber is arranged, which comprises the gas sensor 54. Behind the sensor a pump 53 is installed, which pumps the air collected by the funnel 52 through the sensor cell to the exhaust 55. For conducting the gas, a pipe system is provided connecting the above components. Various gas sensors 54 can be used, but preferred are the same sensors and sensor materials as described above. Even more preferred are sensors as depicted in Fig. 2B, using an encapsulated reference sensor, which is used to compensate baseline drifts due to temperature fluctuations. If the sensor 54 detects a defective battery cell 51 the sensor signal 56 may trigger a robot system 57, which may e.g. sort out the defective battery automatically.

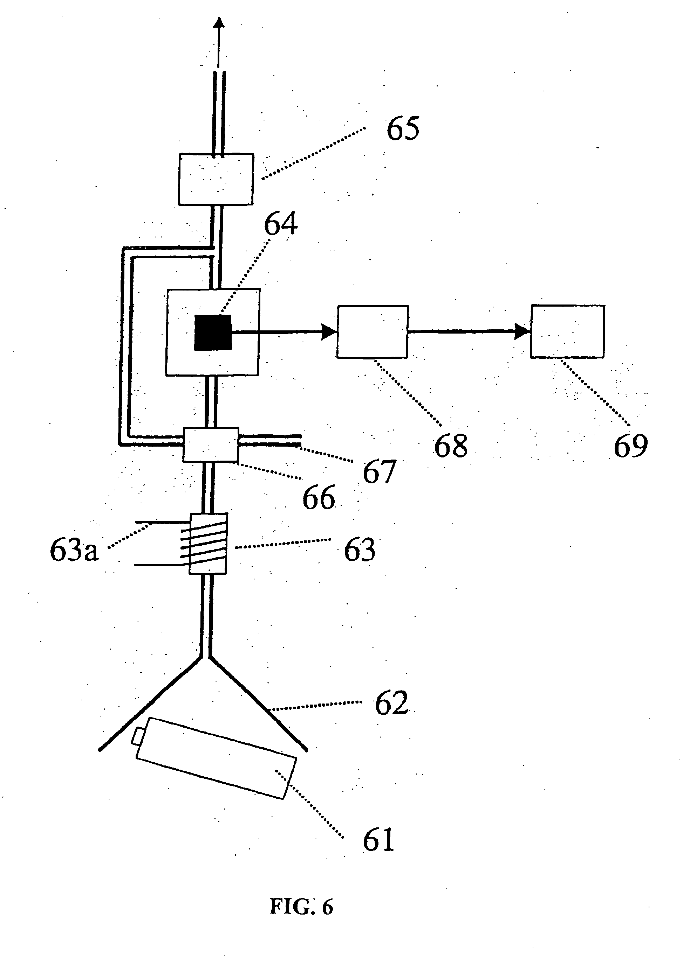

A system according to a preferred embodiment using a pre-concentrator unit is depicted in Fig. 6. To enhance the sensitivity for the detection of a defective battery 51, the sensor system may employ a pre-concentrator unit 63. Pre-concentrator units are commonly known to persons skilled in the art. The pre-concentrator unit 63 is installed in front of the gas sensor 64. Between the two components a four-port valve 66 is provided. In the pre-concentration mode the valve is in a position which allows purging uncontaminated air from inlet 67 through the sensor chamber. During this time the baseline of the sensor 64 is measured. At the same time the air collected by the funnel 62 is pumped with a pump 65 through the pre-concentrator unit 63, where volatile compounds are adsorbed to a suitable adsorbent (e.g. Carbopack X, Tenax TA or Carboxen 1000), such as used in gas chromatography. The pre-concentration procedure is stopped by switching the four-port valve 66 into a position where the pre-concentrator unit 63 is connected with the sensor chamber and the uncontaminated air from the inlet 67 is pumped through the bypass. At the same time, or slightly delayed, the compounds, which may have adsorbed the adsorbent inside the pre-concentrator unit 63 are desorbed by applying a heat pulse with the heater 63a. The released volatile compounds which are now pumped through the sensor chamber and which are getting in contact with the gas sensor 64 trigger a sensor signal 68. As described above the sensor signal may be used to sort out the detected defective battery 61 by means of a system 69. To optimize the system it may comprise further valves or nozzles for optimizing the gas flow. The same preferred sensors and sensor materials as described above may be used.

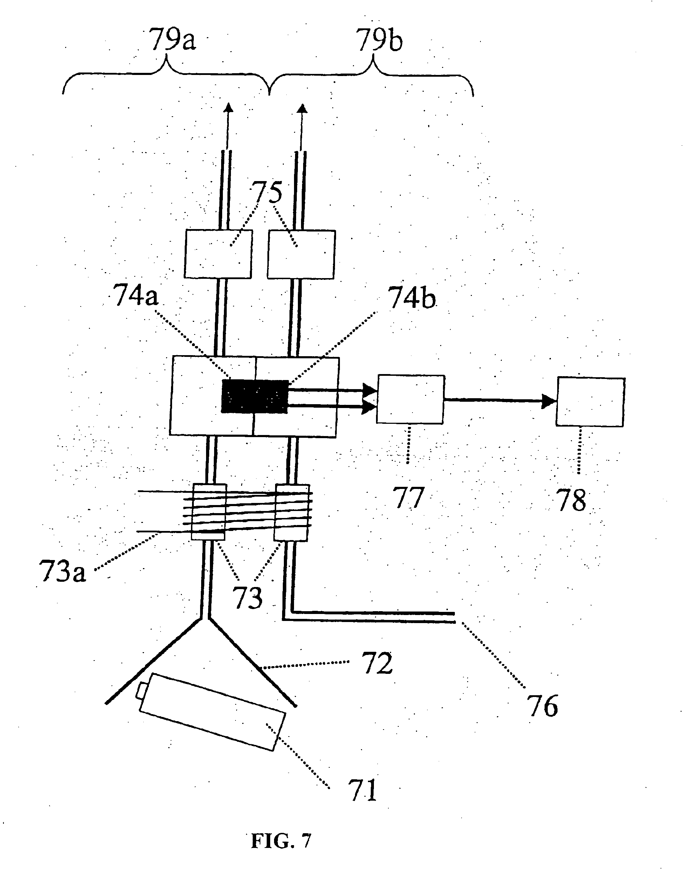

An embodiment according to a more advanced version of the system is shown in Fig. 7. The system is comprised of two pre-concentration units 73 and two sensor chambers containing two sensors 74a and 74b, respectively. One of the systems 79b is used as the reference system. As described above, the sensors 74a, 74b of both systems are preferably in good thermal contact with each other. Both systems work synchronized. In the pre-concentration phase uncontaminated air from the inlet 76 is pumped with the pumps 75 through the pre-concentrator 73 and the sensor chamber of the reference system 79b. At the same time, air collected by the funnel 72 is pumped through the pre-concentrator and the sensor chamber of the sampling system 79a. The pre-concentration phase is stopped by heating both pre-concentrator units 73, by means of coils surrounding the respective pre-concentrator unit 73 and being supplied by wires 73a, to desorb possibly adsorbed chemicals. In case the battery 71 did not leak, both sensor signals 77 are similar and the ratio of the sensor signals should not change significantly. If, however, the battery 71 investigated leaks volatile chemicals which were concentrated in the pre-concentration unit 73 of the sampling system, both sensor signals 77 should differ significantly, and the signal ratio should change. This signal may then be used to sort out a defective battery 71 by means of a suited device 78. To optimize the system it may comprise further valves or nozzles optimizing the gas flow. The system may also be simplified by omitting components such as the pre-concentration unit 73 of the reference system. The same preferred sensors and sensor materials as described above may be used.

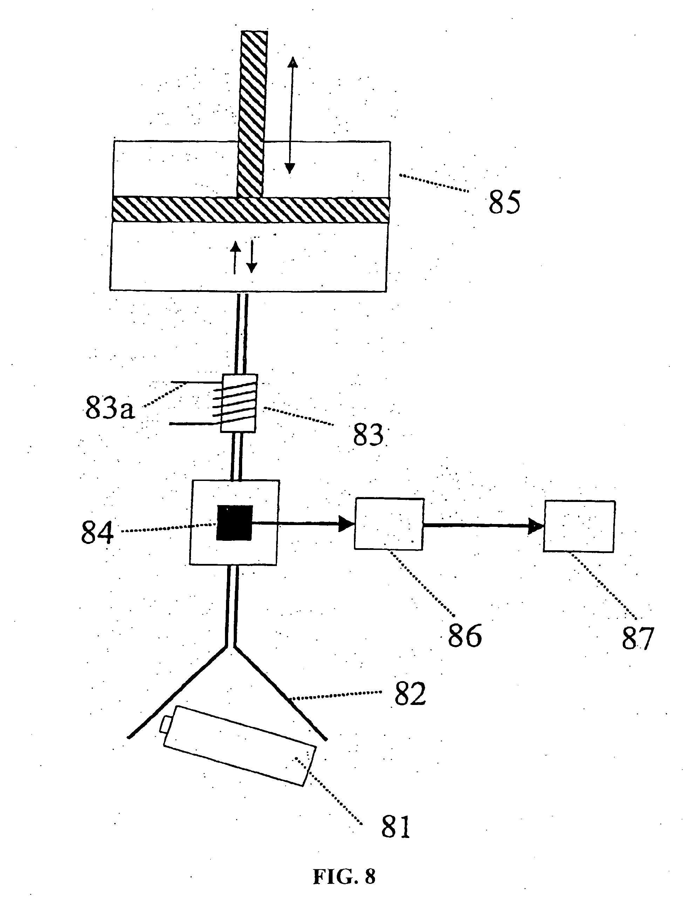

Another preferred embodiment of a detection system according to the invention is shown in Fig. 8. In this example the pump system is a "breathing system" 85. As it draws air from the funnel 82 through the sensor cell, the pre-concentrator unit 83 collects volatile chemicals from a leaking battery cell 81. After switching the direction of the gas flow, the pre-concentrator unit 83 is heated to desorb chemicals from the unit 83. The desorbed chemicals are then detected by the sensor 84 within the sensor chamber. The sensor signal 86 may be used to sort out the defective battery by means of a suited device 87 or may be used for any other purpose such as producing a corresponding indication on an electronic device such as a computer. In analogy to the system depicted in Fig. 7, the system may be equipped with a reference system. The same preferred sensors and sensor materials as described above are preferred.

In order to increase the throughput two or more of any sensor system described above may be combined. The combined sensor systems preferably work in parallel and enable a high throughput of battery cells.

During the quality control procedure the battery cells may be heated above room temperature in order to enhance the evaporation of chemicals from a leaking battery cell.

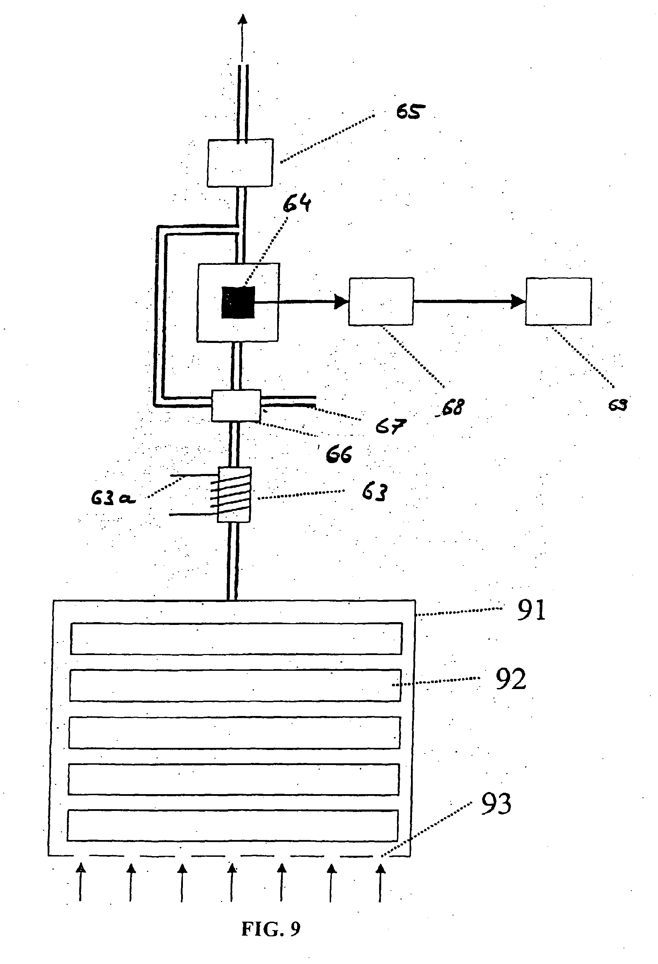

The sensor systems described above may also be used for product control purposes. In such a case it is the goal to detect one or a few defective battery cells in a container with many other intact battery cells. The simplest solution for this application is essentially a larger version of the system shown in Fig. 4 which can contain many batteries. Fig. 9 depicts a battery product control system according to a corresponding embodiment being similar to the embodiment of Fig. 6. In Fig. 9 the same reference numerals are used for the same or similar parts. Instead of a funnel a box 91 is installed containing several batteries 92. Furthermore the box comprises openings 93 for the inlet of air. The same sensor configurations as described above can be used. To enhance the reliability of the system two or more sensors may be installed within the cover or box respectively. Each sensor cover may also use a reference sensor, which may be located inside the cover or outside the cover as explained above. Instead of a cover, which is partly open, it is also possible to use a closed container, which contains the batteries and the sampling sensor.

Since the sample volume is much larger than in the case of quality control of single battery cells, sensor systems, which work with pre-concentrator units can be very useful for product control applications. Thus, in principle the same sensor systems which are combined with a pre-concentrator unit and which are described above can be used. It is preferred that the funnel completely covers a batch of batteries. It is also possible that the sampling system is combined with a box, which contains the batteries and which is equipped with a ventilation system. The ventilation system ensures that the airflow is distributed uniformly in the battery container so that the airflow in the local environment of each battery is about the same.

In parallel to the gas sampling process described above the battery cells may be charged, and/or their electrical performance may be checked. In this case the container is equipped with electrical leads and electrodes to address each battery electrically. During the product control procedure the battery cells may also be heated above room temperature in order to enhance the evaporation of chemicals from a leaking battery cell and to test their performance at various temperatures.

For all these embodiments the use of gas sensors which do not require internal heating - in contrast to most metal oxide based sensors which need to be heated for operation - is preferred. This lowers the power consumption of the device. Preferably the sensors according to this invention are based on conducting or semi-conducting polymers or polymer/carbon black composite films as commonly known to the person skilled in the art in this field. More preferred are sensors employing a metal-nanoparticle/organic composite film as gas sensitive coating. Most preferred are films consisting of metal nanoparticles interlinked with bi- or polyfunctional organic molecules.

These sensitive coatings can be used for many types of gas sensors like QCMs, SAW, Chem-FETs devices or sensors which work on the basis of analyte induced changes of their conductance, capacitance, inductance, dielectric permittivity, polarisation, impedance, heat capacity, or temperature as mentioned above.

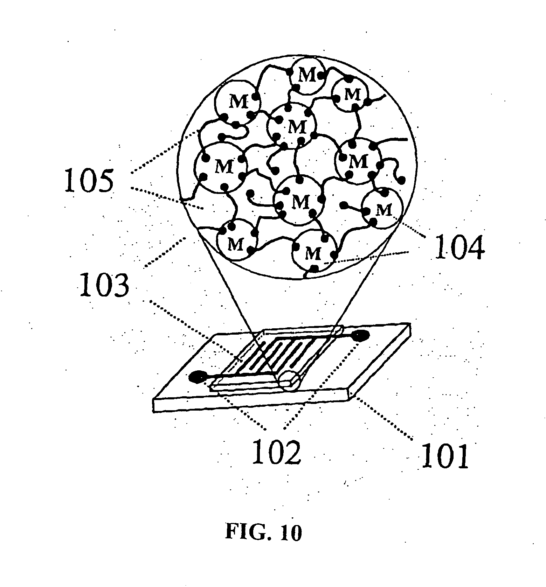

Preferably the change of the conductance should be used to indicate the presence of an analyte, i.e. electrolyte leaking from a defective battery. Besides, the operation of such a chemiresistor in a separate unit also enables an easy integration into integrated circuits. An example for a possible chemiresistor device is shown in Fig. 10. Here a substrate 101 provides an interdigitated electrode structure 102 covered with the chemically sensitive coating 103. This coating is e.g. comprised of metal nanoparticles 104 interlinked with bi- or polyfunctional molecules 105. These coatings can be easily prepared via known layer-by-layer self-assembly methods resulting in homogenous nanoporous thin films. In such films the nanoparticles enable the electrical conduction whereas the organic molecules provide sites for interaction with the analytes. Thus, the selectivity of the sensitive coating can be tuned towards a specified analyte by varying the chemical properties of the organic linker molecules.

The analyte induced change of conductance of such sensor material is usually discussed in terms of swelling of the material and a change of the dielectric environment of the nanoparticle cores as it is known by the person skilled in the art.

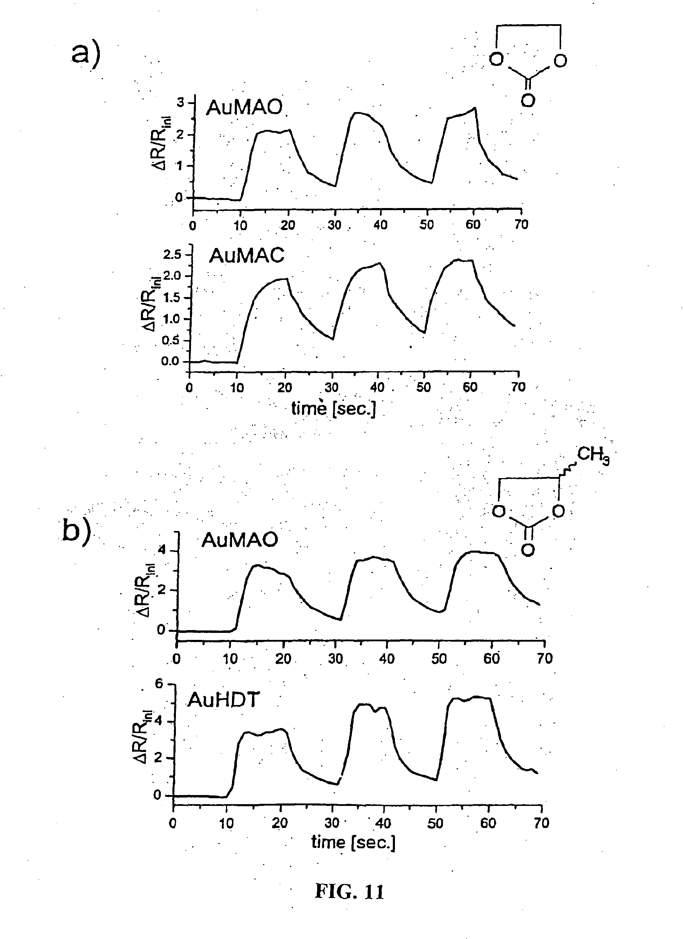

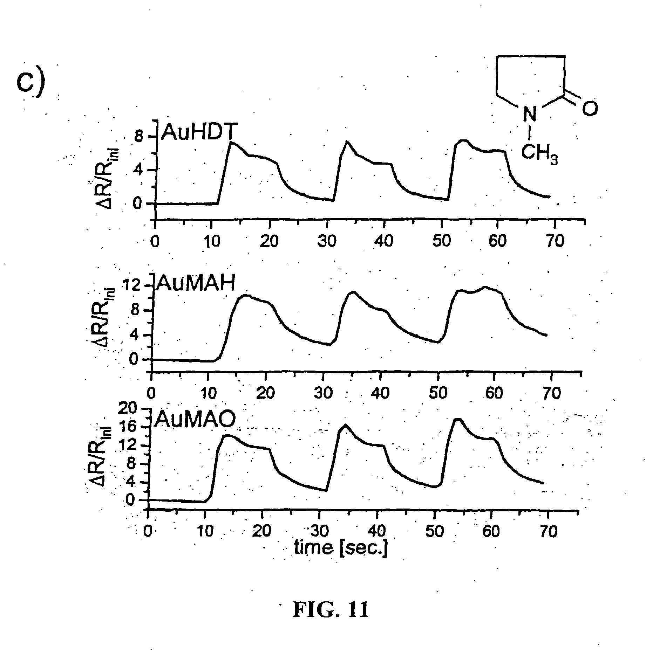

In Fig. 11a)-11c) some sensor responses to vapors of the electrolytes ethylene carbonate (Fig. 11 a), propylene carbonate (Fig. 11b) and the solvent N-methylpropylidinion (Fig. 11c) are shown. In these examples the sensor materials comprise gold nanoparticles interlinked with different organic dithiols (MAO = 1,8-Bis(2-mercaptoacetamido)octane, MAC = 1,4-Bis(2-mercaptoacetamido)cyclohexane, HDT = hexadecane dithiol, MAH = 2,6-Bis(2-mercaptoacetamido)hexane). All sensor materials respond reversibly with an increase in the resistance compared to their initial resistance (ΔR/R_{Ini} = 2-16%) within a few seconds. This result shows that these chemiresistors, which are operated at room temperature, are suited for the purpose of the invention.

Using the following experimental steps the present invention has been realized according to an exemplifying embodiment.
a) Nanoparticle synthesis: These particles were prepared by reduction of AuCl₃ with NaBH₄ in presence of tetraoctylammoniumbromide and dodecylamine as known in prior art. The particles were separated by fractional precipitation. In total 5 fractions were prepared, from which fraction 3 was used for film fabrication. TEM images revealed an average particle diameter of 4 nm and a rather broad size distribution of around 30 %.
b) Synthesis of 1,6-bis(2-mercaptoacetamido)hexane (MAH): 1,6-diaminohexane and triethylamine were stirred with bromacetylbromid. After purification 1,6-bis(bromacetamido)hexane was obtained. The product was stirred with potassiumthioacetate resulting after purification in 1,6-bis(2-thioaceto-acetamido)hexane. This was then cleaved by refluxing with K₂CO₃. After neutralization and purification steps this yields to the desired product: 1,6-bis(2-mercaptoacetamido)hexane (MAH).
c) Synthesis of 1,4-bis(2-mercaptoacetamido)cyclohexane (MAC): For the synthesis of MAC the same route as for MAH was used.
d) Synthesis of 1,8-bis(2-mercaptoacetamido)octane (MAO): For the synthesis of MAO the same route as for MAH was used.
e) Synthesis of 1,16-hexadecanedithiol (HDT): HDT was synthesized according to a commonly known method.
f) Film preparation: The nanoparticle films were prepared using a commonly known layer-by-layer self-assembly method. BK7 glass or oxidized silicon wafers were used as substrates. For investigating the electronic and vapor sensing properties the glass substrates were equipped with interdigitated gold electrode structures (50 finger pairs, 10 µm width and 100 nm height, including a 5 nm titanium adhesion layer, 10 µm spacing, 1800 µm overlap). Prior to film deposition, the substrates were cleaned and functionalized with 3-aminopropyldimethylethoxysilane. After washing the substrates the films were deposited by immersion of the substrates in particle and linker solutions alternately. This was done 10 times for the dendrimers and 14 times for the dithiol linker. Accordingly, the film deposition was finished by treating the substrate with the linker solution, unless otherwise stated. The deposition of the gold particles was monitored by measuring the conductance of the films and collecting UV/vis spectra after each linker exposure. Before such measurements the films were briefly dried under a nitrogen stream.
g) Vapor sensitivity measurement: For investigating the chemical sensitivity of the films the substrates were mounted in a test cell made from teflon. The sensor signal was measured via pogo pins as the relative change of resistance by applying a constant direct current (Keithley Source-Meter 2400) and measuring the voltage (Keithley 2002 Multimeter) across the electrodes whilst switching between air and test vapors. Usually, the sensors were operated with an applied bias of around 0.1 V. As test vapors saturated vapors of ethylene carbonate, propylene carbonate and N-methylpyrolidinon were used. The flow in the test chamber was kept constant for all experiments. All experiments were carried out at room temperature.

The features of the invention disclosed in the claims, in the description and in the drawings may be significant for the realization of the invention either alone or in any combination thereof.

## Claims

1. Battery leakage detection system **characterized in that** the system comprises a gas sensor (13; 24, 25; 35, 36; 42; 64; 74a, 74b; 84; 97) having a gas sensitive nanoparticle structure (23; 103), comprising metal nanoparticles (104) interlinked with bi- or polyfunctional organic molecules (105).

2. System according to claim 1, **characterized in that** the gas sensitive nanoparticle structure (23; 103) is a combination of a metal-nanoparticle/organic composite structure and at least one of a semi-conducting polymer structure or a polymer/carbon black composite structure.

3. System according to one of the preceding claims, **characterized in that** the gas sensor (13; 24, 25; 35, 36; 42; 64; 74a, 74b; 84; 97) is a sensor working on the basis of analyte induced changes of its conductance, capacitance, inductance, dielectric permittivity, polarization, impedance, heat capacity or temperature.

4. System according to one of the preceding claims, **characterized in that** the gas sensor is a mass sensitive gas sensor (13; 24, 25; 35, 36; 42; 64; 74a, 74b; 84; 97), in particular a sensor comprising a quartz crystal microbalance, a surface acoustic wave device or a chemically sensitive field effect transistor.

5. System according to one of the preceding claims, **characterized in that** it comprises at least one reference sensor (25) for said sensor (13; 24; 35, 36; 42; 64; 74a, 74b; 84; 97), said reference sensor (25) and said sensor (13; 24; 35, 36; 42; 64; 74a, 74b; 84; 97) comprising respective gas sensitive structures (23; 103) being isolated from each other.

6. System according to claim 5, **characterized in that** said reference sensor (25) and said sensor (13; 24; 35, 36; 42; 64; 74a, 74b; 84; 97) are in contact for temperature exchange.

7. System according to one of the preceding claims, **characterized in that** it comprises a closed or tight housing (12; 33; 43), in particular a battery housing in which a gas sensor (13; 24, 25; 35, 36; 42; 64; 74a, 74b; 84; 97) is arranged.

8. System according to claim 7, **characterized in that** it comprises a further closed or tight housing (12; 33; 43) in which a further gas sensor (13; 24, 25; 35, 36; 42; 64; 74a, 74b; 84; 97) is arranged.

9. System according to claim 8, **characterized in that** one sensor (13; 24, 25; 35, 36; 42; 64; 74a, 74b; 84; 97) arranged in said housing (12; 33; 43) is a reference sensor for the gas sensor (13; 24, 25; 35, 36; 42; 64; 74a, 74b; 84; 97) in said further housing (12; 33; 43).

10. System according to one of the preceding claims, **characterized in that** it comprises a funnel (52; 62; 72; 82) for collecting volatile chemicals from a defective battery (11; 34; 41; 51; 61; 71; 81; 92), a sensor chamber housing said sensor (13; 24, 25; 35, 36; 42; 64; 74a, 74b; 84; 97), a pump (53; 65; 75; 94) for pumping air to and/or drawing air past said sensor (13; 24, 25; 35, 36; 42; 64; 74a, 74b; 84; 97), and/or a pre-concentrator unit (63; 73; 83; 95) connected to each other.

11. System according to one of the preceding claims **characterized in that** it comprises a means for conveying batteries (11; 34; 41; 51; 61; 71; 81; 92) to and from a test location provided in the system and/or a means for automatically sorting out defective batteries (11; 34; 41; 51; 61; 71; 81; 92).

12. Electrical equipment comprising a system according to one of claims 1 to 11.

13. Method for detecting a leakage of a battery (11; 34; 41; 51; 61; 71; 81; 92) comprising the steps of:
providing a gas sensor (13; 24, 25; 35, 36; 42; 64; 74a, 74b; 84; 97) having a gas sensitive nanoparticle structure (23; 103), comprising metal nanoparticles (104) interlinked with bi- or polyfunctional organic molecules (105), close to a battery (11; 34; 41; 51; 61; 71; 81; 92);
detecting analyte induced changes of the electrical conductance, capacitance, inductance, dielectric permittivity, polarization, impedance, heat capacity or temperature in said gas sensor (13; 24, 25; 35, 36; 42; 64; 74a, 74b; 84; 97) indicating a defective battery (11; 34; 41; 51; 61; 71; 81; 92).

14. Method according to claim 13, **characterized by** the further steps of:
providing a pre-conceritrator unit (63; 73; 83; 95) in front of said gas sensor (13; 24, 25; 35, 36; 42; 64; 74a, 74b; 84; 97);
bringing volatile chemicals from a defective battery (11; 34; 41; 51; 61; 71; 81; 92) in contact with said pre-concentrator unit (63; 73; 83; 95);
applying a heat pulse to said pre-concentrator unit (63; 73; 83; 95) for desorbing volatile chemical compounds adsorbed to said pre-concentrator unit (63; 73; 83; 95); bringing said desorbed volatile chemical compounds in contact with said gas sensor (13; 24, 25; 35, 36; 42; 64; 74a, 74b; 84; 97).

15. Method according to claim 13 or 14, **characterized by** the further step of triggering an optical, acoustical and/or data signal in case an analyte induced change of the electrical conductance, capacitance, inductance, dielectric permittivity, polarization, impedance, heat capacity or temperature in said gas sensor (13; 24, 25; 35, 36; 42; 64; 74a, 74b; 84; 97) is detected.

16. Method according to one of preceding claims, **characterized by** the further step of automatically sorting out said defective battery (11; 34; 41; 51; 61; 71; 81; 92).

## Patentansprüche

1. System zum Nachweis eines Lecks in einer Batterie, **dadurch gekennzeichnet, dass** das System einen Gassensor (13; 24, 25; 35, 36; 42; 64; 74a, 74b; 84; 97) mit einer gassensitiven Nanopartikelstruktur (23; 103) aufweist, die mit bi- oder polyfunktionalen organischen Molekülen (105) verbundene Metallnanopartikel (104) aufweist.

2. System nach Anspruch 1, **dadurch gekennzeichnet, dass** die gassensitive Nanopartikelstruktur (23; 103) eine Kombination aus einer Metallnanopartikel/organischen Verbundstruktur und mindestens einem von einer halbleitenden Polymerstruktur oder einer Polymer/Carbon-Black-Verbundstruktur ist.

3. System nach einem der vorhergehenden Ansprüche, **dadurch gekennzeichnet, dass** der Gassensor (13; 24, 25; 35, 36; 42; 64; 74a, 74b; 84; 97) ein Sensor ist, der auf der Basis von Analyt-induzierten Änderungen seiner Leitfähigkeit, Kapazität, Induktivität, dielektrischen Permittivität, Polarisation, Impedanz, Wärmekapazität oder Temperatur arbeitet.

4. System nach einem der vorhergehenden Ansprüche, **dadurch gekennzeichnet, dass** der Gassensor ein massesensitiver Gassensor (13; 24, 25; 35, 36; 42; 64; 74a, 74b; 84; 97) ist, insbesondere ein Sensor, der eine Quarzkristallmikrowaage, eine akustische Oberflächenwellenvorrichtung oder einen chemisch sensitiven Feldeffekttransistor aufweist.

5. System nach einem der vorhergehenden Ansprüche, **dadurch gekennzeichnet, dass** es mindestens einen Referenzsensor (25) für den Sensor (13; 24; 35, 36; 42; 64; 74a, 74b; 84; 97) aufweist, wobei der Referenzsensor (25) und der Sensor (13; 24; 35, 36; 42; 64; 74a, 74b; 84; 97) entsprechende gassensitive Strukturen (23; 103) aufweisen, die voneinander isoliert sind.

6. System nach Anspruch 5, **dadurch gekennzeichnet, dass** der Referenzsensor (25) und der Sensor (13; 24; 35, 36; 42; 64; 74a, 74b; 84; 97) zum Austausch von Temperatur in Kontakt sind.

7. System nach einem der vorhergehenden Ansprüche, **dadurch gekennzeichnet, dass** es ein verschlossenes oder dichtes Gehäuse (12; 33; 43) aufweist, insbesondere ein Batteriegehäuse, in dem ein Gassensor (13; 24, 25; 35, 36; 42; 64; 74a, 74b; 84; 97) angeordnet ist.

8. System nach Anspruch 7, **dadurch gekennzeichnet, dass** es ein weiteres geschlossenes oder dichtes Gehäuse (12; 33; 43) aufweist, in dem ein weiterer Gassensor (13; 24, 25; 35, 36; 42; 64; 74a, 74b; 84; 97) angeordnet ist.

9. System nach Anspruch 8, **dadurch gekennzeichnet, dass** ein Sensor (13; 24, 25; 35, 36; 42; 64; 74a, 74b; 84; 97), der in dem Gehäuse (12; 33; 43) angeordnet ist, ein Referenzsensor für den Gassensor (13; 24, 25; 35, 36; 42; 64; 74a, 74b; 84; 97) in dem weiteren Gehäuse (12; 33; 43) ist.

10. System nach einem der vorhergehenden Ansprüche, **dadurch gekennzeichnet, dass** es einen Trichter (52; 62; 72; 82) zum Sammeln von flüchtigen Chemikalien von einer fehlerhaften Batterie (11; 34; 41; 51; 61; 71; 81; 92), eine Sensorkammer, die den Sensor (13; 24, 25; 35, 36; 42; 64; 74a, 74b; 84; 97) aufnimmt, eine Pumpe (53; 65; 75; 94) zum Pumpen von Luft zu und/oder zum Saugen von Luft vorbei an dem Sensor (13; 24, 25; 35, 36; 42; 64; 74a, 74b; 84; 97) und/oder eine Präkonzentratoreinheit (63; 73; 83; 95) aufweist, die miteinander verbunden sind.

11. System nach einem der vorhergehenden Ansprüche, **dadurch gekennzeichnet, dass** es ein Mittel zum Befördern von Batterien (11; 34; 41; 51; 61; 71; 81; 92) zu und von einer Prüfstelle, die im System bereitgestellt ist, und/oder ein Mittel zum automatischen Aussortieren von fehlerhaften Batterien (11; 34; 41; 51; 61; 71; 81; 92) aufweist.

12. Elektrische Einrichtung, die ein System nach einem der Ansprüche 1 bis 11 aufweist.

13. Verfahren zum Erfassen eines Lecks einer Batterie (11; 34; 41; 51; 61; 71; 81; 92), das die folgenden Schritte aufweist:
Bereitstellen eines Gassensors (13; 24, 25; 35, 36; 42; 64; 74a, 74b; 84; 97) mit einer gassensitiven Nanopartikelstruktur (23; 103), die mit bi- oder polyfunktionalen organischen Molekülen (105) verbundene Metallnanopartikel (104) aufweist, nahe an einer Batterie (11; 34; 41; 51; 61; 71; 81; 92);
Erfassen von Analyt-induzierten Änderungen der elektrischen Leitfähigkeit, Kapazität, Induktivität, dielektrischen Permittivität, Polarisation, Impedanz, Wärmekapazität oder Temperatur im Gassensor (13; 24, 25; 35, 36; 42; 64; 74a, 74b; 84; 97), die eine fehlerhafte Batterie (11; 34; 41; 51; 61; 71; 81; 92) anzeigen.

14. Verfahren nach Anspruch 13, **gekennzeichnet durch** die folgenden weiteren Schritte:
Bereitstellen einer Präkonzentratoreinheit (63; 73; 83; 95) vor dem Gassensor (13; 24, 25; 35, 36; 42; 64; 74a, 74b; 84; 97);
In-Kontakt-Bringen von flüchtigen Chemikalien von einer fehlerhaften Batterie (11; 34; 41; 51; 61; 71; 81; 92) mit der Präkonzentratoreinheit (63; 73; 83; 95);
Anwenden eines Wärmeimpulses auf die Präkonzentratoreinheit (63; 73; 83; 95) zum Desorbieren von flüchtigen chemischen Verbindungen, die an die Präkonzentratoreinheit (63; 73; 83; 95) adsorbiert sind;
In-Kontakt-Bringen der desorbierten flüchtigen chemischen Verbindungen mit dem Gassensor (13; 24, 25; 35, 36; 42; 64; 74a, 74b; 84; 97).

15. Verfahren nach Anspruch 13 oder 14, **gekennzeichnet durch** den weiteren Schritt eines Auslösens eines optischen, akustischen Signals und/oder Datensignals in dem Fall, dass eine Analyt-induzierte Änderung der elektrischen Leitfähigkeit, Kapazität, Induktivität, dielektrischen Permittivität, Polarisation, Impedanz, Wärmekapazität oder Temperatur im Gassensor (13; 24, 25; 35, 36; 42; 64; 74a, 74b; 84; 97) erfasst wird.

16. Verfahren nach einem der vorhergehenden Ansprüche, **gekennzeichnet durch** den weiteren Schritt eines automatischen Aussortierens der fehlerhaften Batterie (11; 34; 41; 51; 61; 71; 81; 92).

## Revendications

1. Système de détection de fuite dans une batterie, **caractérisé en ce que** le système comprend un détecteur de gaz (13 ; 24, 25 ; 35, 36 ; 42 ; 64 ; 74a, 74b ; 84 ; 97) ayant une structure de nanoparticules sensibles au gaz (23 ; 103) comprenant des nanoparticules de métal (104) enchaînées avec des molécules organiques bifonctionnelles ou polyfonctionnelles (105).

2. Système selon la revendication 1, **caractérisé en ce que** la structure de nanoparticules sensibles au gaz (23 ; 103) est une combinaison d'une structure composite nanoparticules de métal/organique et d'au moins une structure parmi une structure en polymère semi-conducteur et une structure composite polymère/noir de carbone.

3. Système selon l'une des revendications précédentes, **caractérisé en ce que** le détecteur de gaz (13 ; 24, 25 ; 35, 36 ; 42 ; 64 ; 74a, 74b ; 84 ; 97) est un détecteur fonctionnant sur la base de changements, induits par un analyte, de sa conductivité, sa capacité, son inductance, sa permissivité diélectrique, sa polarisation, son impédance, sa capacité calorifique ou sa température.

4. Système selon l'une des revendications précédentes, **caractérisé en ce que** le détecteur de gaz est un détecteur de gaz sensible à la masse (13 ; 24, 25 ; 35, 36 ; 42 ; 64 ; 74a, 74b ; 84 ; 97), en particulier un détecteur comprenant une microbalance à cristal de quartz, un dispositif à ondes acoustiques de surface, ou un transistor à effet de champ sensible sur le plan chimique.

5. Système selon l'une des revendications précédentes, **caractérisé en ce qu'**il comprend au moins un détecteur de référence (25) pour ledit détecteur (13 ; 24, 25 ; 35, 36 ; 42 ; 64 ; 74a, 74b ; 84 ; 97), ledit détecteur de référence (25) et ledit détecteur (13 ; 24, 25 ; 35, 36 ; 42 ; 64 ; 74a, 74b ; 84 ; 97) comprenant des structures respectives sensibles au gaz (23 ; 103) qui sont isolées les unes des autres.

6. Système selon la revendication 5, **caractérisé en ce que** ledit détecteur de référence (25) et ledit détecteur (13 ; 24, 25 ; 35, 36 ; 42 ; 64 ; 74a, 74b ; 84 ; 97) sont en contact pour un échange de température.

7. Système selon l'une des revendications précédentes, **caractérisé en ce qu'**il comprend un boîtier fermé ou étanche (12 ; 33 ; 44) en particulier un boîtier de batterie dans lequel est agencé un détecteur de gaz (13 ; 24, 25 ; 35, 36 ; 42 ; 64 ; 74a, 74b ; 84 ; 97).

8. Système selon la revendication 7, **caractérisé en ce qu'**il comprend un autre boîtier fermé ou étanche (12 ; 33 ; 43) dans lequel est agencé un autre détecteur de gaz (13 ; 24, 25 ; 35, 36 ; 42 ; 64 ; 74a, 74b ; 84 ; 97).

9. Système selon la revendication 8, **caractérisé en ce qu'**un détecteur (13 ; 24, 25 ; 35, 36 ; 42 ; 64 ; 74a, 74b ; 84 ; 97) agencé dans ledit boîtier (12 ; 33 ; 43) est un détecteur de référence pour le détecteur de gaz (13 ; 24, 25 ; 35, 36 ; 42 ; 64 ; 74a, 74b ; 84 ; 97) dans ledit autre boîtier (12 ; 33 ; 43).

10. Système selon l'une des revendications précédentes, **caractérisé en ce qu'**il comprend un entonnoir (52 ; 62 ; 72 ; 82) pour collecter des produits chimiques volatils provenant d'une batterie défectueuse (11 ; 31 ; 41 ; 51 ; 61 ; 71 ; 81 ; 92), une chambre de détecteur abritant ledit détecteur (13 ; 24, 25 ; 35, 36 ; 42 ; 64 ; 74a, 74b ; 84 ; 97), une pompe (53 ; 65 ; 75 ; 94) pour pomper de l'air vers ledit détecteur et/ou aspirer de l'air en passant devant ledit détecteur (13 ; 24, 25 ; 35, 36 ; 42 ; 64 ; 74a, 74b ; 84 ; 97), et/ou une unité de préconcentration (63 ; 73 ; 83 ; 95) connectées les unes aux autres.

11. Système selon l'une des revendications précédentes, **caractérisé en ce qu'**il comprend des moyens pour convoyer des batteries (11 ; 34 ; 41 ; 51 ; 61 ; 71 ; 81 ; 92) vers un emplacement de test ou depuis celui-ci, prévu dans le système, et/ou des moyens pour trier automatiquement les batteries défectueuses (11 ; 34 ; 41 ; 51 ; 61 ; 71 ; 81 ; 92).

12. Équipement électrique comprenant un système selon l'une des revendications 1 à 11.

13. Procédé pour détecter une fuite d'une batterie (11 ; 34 ; 41 ; 51 ; 61 ; 71 ; 81 ; 92) comprenant les étapes consistant à :
fournir un détecteur de gaz (13 ; 24, 25 ; 35, 36 ; 42 ; 64 ; 74a, 74b ; 84 ; 97) ayant une structure de nanoparticules sensibles au gaz (23 ; 103) comprenant des nanoparticules de métal (104) enchaînées avec des molécules organiques (105) bifonctionnelles ou polyfonctionnelles, à proximité d'une batterie (11 ; 34 ; 41 ; 51 ; 61 ; 71 ; 81 ; 92) ;
détecter des changements, induits par un analyte, de la conductivité électrique, la capacité, l'inductance, la permissivité diélectrique, la polarisation, l'impédance, la capacité thermique ou la température dans ledit capteur de gaz (13 ; 24, 25 ; 35, 36 ; 42 ; 64 ; 74a, 74b ; 84 ; 97) indiquant une batterie défectueuse (11 ; 34 ; 41 ; 51 ; 61 ; 71 ; 81 ; 92).

14. Procédé selon la revendication 13, **caractérisé par** les autres étapes consistant à :
fournir une unité de préconcentration (63 ; 73 ; 83 ; 95) en face dudit détecteur de gaz (13 ; 24, 25 ; 35, 36 ; 42 ; 64 ; 74a, 74b ; 84 ; 97) ;
amener des produits chimiques volatils provenant d'une batterie défectueuse (11 ; 34 ; 41 ; 51 ; 61 ; 71 ; 81 ; 92) en contact avec ladite unité de préconcentration (63 ; 73 ; 83 ; 95) ;
appliquer une impulsion thermique à ladite unité de préconcentration (63 ; 73 ; 83 ; 95) pour désorber des composés chimiques volatils adsorbés sur ladite unité de préconcentration (63 ; 73 ; 83 ; 95) ;
amener lesdits composés chimiques volatils désorbés en contact avec ledit détecteur de gaz (13 ; 24, 25 ; 35, 36 ; 42 ; 64 ; 74a, 74b ; 84 ; 97).

15. Procédé selon la revendication 13 ou 14, **caractérisé par** l'étape supplémentaire consistant à déclencher un signal optique, acoustique et/ou de donner dans le cas où un changement, induit par un analyte, de la conductivité électrique, la capacité, l'inductance, la permissivité diélectrique, la polarisation, l'impédance, la capacité thermique ou la température dans ledit détecteur de gaz (13 ; 24, 25 ; 35, 36 ; 42 ; 64 ; 74a, 74b ; 84 ; 97) est détecté.

16. Procédé selon l'une des revendications précédentes, **caractérisé par** l'étape supplémentaire consistant à trier automatiquement lesdites batteries défectueuses (11 ; 34 ; 41 ; 51 ; 61 ; 71 ; 81 ; 92).
